# EUROPEAN PATENT APPLICATION

(11) **EP 1 972 929 A1**
(43) Date of publication of application: **24.09.2008**
(21) Application number: 08003603.1
(22) Date of filing: 27.02.2008
(51) Int. Cl.: G01N 21/78

(54) **Dry optical - chemical carbon - dioxide sensor**

(30) Priority: 27.02.2007 EP 07004082
(71) Applicant: F. Hoffmann-La Roche AG, 4070 Basel (CH); Roche Diagnostics GmbH, 68305 Mannheim (DE); PreSens Precision Sensing GmbH, 86633 Neuburg a.d. Donau (DE)
(72) Inventor: Leiner, Marco Jean Pierre, 8045 Graz (AT); Tusa, James Kenneth, Georgia 30004 (US); Klimant, Ingo, 8200 Hintersberg (AT)
(74) Representative: Weiss, Wolfgang

(57) **Abstract**

A device for determining carbon dioxide in a gaseous or liquid sample, comprises: a polymer matrix e.g. ethyl cellulose, polysiloxanes etc. and an indicator embedded in the polymer matrix, wherein the indicator comprises a pH-sensitive dye e.g. bromothymol blue, HPTS etc. and a lipophilic, metal cation-ionophore complex e.g. K-18-crown-6, and wherein an anion of the pH-sensitive dye and the metal cation-ionophore complex form an ion-pair which is soluble in the polymer matrix.

## Description

The present invention relates to a device and a method for determining carbon-dioxide (CO₂) in gaseous or liquid samples. Particularly, the invention relates to a device for determining the partial pressure of carbon dioxide.

The measurement of CO₂ in gaseous samples or liquids may be inferred from a pH measurement. Several devices for measuring CO₂ or other acidic gases or liquids are known in the art.

An optical-chemical sensor (also designated as optode or optrode) comprises a sensor matrix, which is a composition comprising an indicator whose optical properties vary with the concentration of a particular analyte contained in a sample. The sensor matrix typically consists of one or more compositions of inorganic and/or organic, preferably polymeric, substances which may be applied on a transparent carrier or substrate, with at least one composition containing the indicator. The carrier may be planar, cylindrical, or of any other shape. For example the compositions are layers which may be applied to the "wells" of micro-titration plates, at the tip of optical fibre bundles or on single optical fibres or light-guiding structures. An optical-chemical sensor is usually able to measure reversibly and often continuously. Exceptions to this rule are for example certain enzyme-carrying biochemical sensors. An optical-chemical sensor may be placed in contact with the sample and, when exposed to light, provides optically readable information about a particular analyte of interest which is present in the sample (e.g. concentration, activity or partial pressure).

According to the present invention an "optical chemical carbon-dioxide sensor" encompasses a sensor matrix, which is a composition comprising the indicator or a composition in which the indicator is embedded. The term "sensor" refers to the interface between the sample medium and components of a measuring device; in particular, it refers to one or more layers or other forms of inorganic and/or organic, preferably polymeric, substances applied on a transparent carrier or substrate, with at least one layer containing an indicator or indicator system whose optical characteristics (absorption, luminescence, luminescence decay time and/or luminescence polarisation) vary with the concentration or partial pressure of carbon-dioxide contained in a sample. The indicator may be homogeneously distributed within the sensor matrix or at least one layer thereof. Alternatively the indicator may be present in heterogeneous form, e.g. in form of particles which are dispersed within the sensor matrix or at least one layer thereof.

The components of the measuring system or the measuring device, may comprise optical and electronic components such as light source, detector, optical filters, electronic signal amplifiers and the evaluation unit. These components are not part of the optical sensor.

An optical chemical CO₂ sensor useful for the determination of CO₂ partial pressure of blood is e.g. disclosed in US 5,496,521, Sensors and Actuators B29, 169-73 (1995); and Ann. Biol. Clin. 61, 183-91, (2003). The sensor is a Severinghaus-type sensor that requires the presence of a pH-buffer solution to maintain the internal pH at predetermined value depending on the external partial pressure of CO₂. The buffer is typically made of some salts of weak acids, such as bicarbonate, phosphate, or HEPES etc., present in a porous layer or an hydrophilic polymer or the like. A disadvantage of the traditional Severinghaus-type sensors is that they take time to equilibrate with water and therefore have to be stored under wet conditions. Further, the salts may precipitate when the sensor becomes dry which causes problems for fast and rapid wet-up. The concentration of the salts depends on moisture content of gaseous samples and osmotic pressure of liquid samples, respectively, which causes severe problems of sensor's response and stability.

An approach for determining carbon dioxide in gases with optical-chemical sensors in which impregnated porous or otherwise adsorbent surfaces change their optical properties reversibly in contact with CO₂ containing gases, is known from Guenther et al. (US 3,694,164 and US 3,754,867). In one embodiment, the composition of the sensing element includes a porous carrier, such as filter paper or a gelled film, phenol red, potassium hydroxide and triethylene glycol or other water-miscible glycols. The sensor may also comprise a protective polymeric film. Because of the hygroscopic nature of the components employed in the sensing layer such sensor is only useful for sensing CO₂ in gases. To improve and enhance the response of the dye, Raemer et al., (US 5 005 572) disclose a mixture of a pH sensitive dye and a phase transport enhancer, both added to a solid phase support.

A further similar approach to optical carbon dioxide sensing known in the art is based on the concept of 'dry' plastic film CO₂ sensors, proposed by Mills and co-workers (Anal. Chem., 1992, 64, 1383, US 5,472,668 and US 5,480,611). The approach makes use of ion pairs consisting of a pH indicator dye anion (Dye-) and an organic quaternary ammonium cation (Q ⁺).The resulting 'dry' (as opposed to the buffer-base 'wet' sensor chemistry) or 'plastic' sensor membrane comprises a pH indicator dye immobilized along with a quaternary ammonium cation, and an amount of an organic quaternary ammonium hydroxide (Q⁺ OH) within a polymer layer on a suitable support. Such sensors are applicable to optical sensing of carbon dioxide both in dry gases, where they have usually short response times, and in liquid samples. Typically, the sensor matrix consists of an water-immiscible, essentially non-polar polymer with or without plasticizer. Further, the sensor matrix may comprise a small number of molecules of water of solvation associated with the ionic compounds present in the matrix. Thus, the principle of this approach is to incorporate an organic cationic substance with an appropriate hydrophobic organic plasticizer and an organic dye into a layer (plastic film) consisting of an essentially non-polar polymer. The final layer (plastic film) has the properties of an organic liquid phase.

The quaternary ammonium hydroxide is added to
(a) improve the solubility of the negatively charged dye components in the polymer layer by forming organic soluble ion-pairs, .
(b) provide the matrix an initial basic environment crucial to sensor functioning, and
(c) offer an additional means of adjusting the hydrophobicity of the dye layer.

Such sensor possesses excellent properties, including fast response/recovery, typically in the range of sub-second to seconds in response to gaseous CO₂ good homogeneity in dry film and simplicity in design because no watery liquid is involved.

A drawback of the 'plastic film' sensors is, however, that the shelf life is not long, typically within days or a few weeks in an open atmosphere, or a few months in a sealed container. During the storage, the baseline and slope of the sensor drifts, and therefore frequent calibration is required prior to use. The shelf instability of the sensor is the result of a gradual loss of the organic base, which may in turn be the results of two things, namely the sensor is "poisoned" by some acidic species, and/or the base itself undergoes, especially at elevated temperature, chemical degradation known as Hofmann beta-hydrogen elimination reaction (Mills et al., Anal. Chem. 64 (1992), 1388, col. 2, para 485; Mills and Monaf, Analyst 121 (1996), pg. 539 col. 2, line 15 ff.; Weigl and Wolfbeis, Sensors and Actuators B28 (1995), pg. 155, col. 1, section 2 and Waldner et al., US 6,338,822, col. 12, I. 64 - col. 13, I. 41).

EP 0 837 327 proposes both Severinghaus-type and Mills-type optical carbon dioxide sensors using so-called Fluorescence Resonance Energy Transfer (FRET; cf. *infra)* indicator dye systems. With reference to EP 0 105 870, EP 0 837 327 discloses FRET-type CO₂-sensors using ion pairs comprising an anionic pH-sensitive chromophore and a cationic pH-insensitive luminophore in an aqueous micro-droplet environment dispersed within an ion-impermeable, gas-permeable polymer matrix. With reference to Mills et al., Anal. Chem. 64 (1992), 1383-1389, EP 0 837 327 discloses Mills-type FRET-CO₂-sensors, some of them even substituting the Mills-typical quaternary ammonium cation Q⁺ with a cationic luminophore, e. g., Ruthenium (II) and Osmium (II) complexes. For a FRET system, substitution of the optically inactive cationic substance [Q+] by an optically active substance, namely a cationic luminophore acting as luminescent donor dye, has a substantial disadvantage. The radiation-less energy-transfer will not take place if the donor and acceptor molecules are not in close spatial proximity. The latter will likely be the case for luminophore molecules not ion-paired with the pH-sensitive chromophore. Moreover, preferred sensor compositions require a molar ratio of dye and cationic species which is by far greater than 1:1. Consequently, substitution of the optically inactive cationic substance [Q+] by a cationic luminophore will reveal an unfeasible sensor composition because of an unacceptable high baseline.

A comprehensive overview over both wet and dry optical carbon dioxide sensors is given by A. Mills and S. Hodgen, ,Fluorescent Carbon Dioxide Sensors' in ,Topics in Fluorescence Spectroscopy', Volume 9, Advanced Concepts in Fluorescence Sensing, Part A: Small Molecule Sensing, C. D. Geddes and J. R. Lakowicz (eds.), Springer, 2005, p. 119-161.

Thus, it was an object of the present invention to provide an optical sensor and a sensing device for determining CO₂ in which the disadvantages of the prior art are at least partially overcome. One object of the invention was to provide a CO₂ sensor with increased shelf stability. A particular object of the present invention was to provide a CO2 sensor using cationic species characterized by improved chemical stability.

The present invention refers to a device for determining CO₂ in a gaseous or liquid sample, comprising a matrix comprising a polymer and optionally a plasticizer, an indicator, and a cationic species comprising a metal cation-ionophore complex dispersed in the matrix, wherein the indicator comprises a pH-sensitive dye or a pH-sensitive dye system, which can form an anionic dye species, and wherein the anionic dye species and the cationic species can form an ion pair which is soluble in the matrix.

In particular, the present invention refers to an optical sensor for determining carbon dioxide in a gaseous or liquid sample, the sensor comprising:
a matrix comprising a preferably homogeneous mixture of
   (a) a polymer,
   (b) optionally a plasticizer,
   (c) an indicator comprising a pH-sensitive dye which can form an anionic dye species,
   (d) a cationic species, and
   (e) an anionic species which is basic in relation to the protonated form of the pH-sensitive dye,
wherein the cationic species is a lipophilic metal cation-ionophore complex.

Another aspect of the present invention is directed to an optical sensor for determining carbon dioxide in a gaseous or liquid sample, comprising:
a matrix comprising a mixture of
   (a) a polymer,
   (b) optionally a plasticizer,
   (c) an indicator comprising a pH-sensitive dye which can form an anionic dye species,
   (d) a cationic species, and
   (e) an anionic species which is basic in relation to the protonated form of the pH-sensitive dye,
wherein the cationic species is a lipophilic, metal cation-ionophore complex and wherein the cationic species acts as a counterion for the anionic dye species.

Depending on the type of dye or dye-system the response caused by the analyte concentration or partial pressure may be affected by very different photophysical mechanisms. In the following, examples of preferred dyes and dye systems are indicated.

The dye may be a substance whose absorbance response (e.g. light absorption), depends on the concentration or partial pressure of CO₂ via direct or indirect interaction, e.g. an absorbance dye, a photometric dye or a colorimetric dye.

The dye may also be a substance whose luminescent response (e.g. luminescence intensity, and/or luminescence decay time) depends on the concentration or partial pressure of carbon dioxide via direct or indirect interaction, e.g. fluorescent or phosphorescent dye.

The present invention preferentially relates to luminescence-optical sensors. Such sensors contain at least one luminescent dye, e.g. in at least one layer of the sensor matrix.

The dye may also comprise a FRET indicator dye system (FRET = Fluorescence Resonance Energy Transfer) which comprises two dyes, a luminescent donor dye and an acceptor dye. The luminescence of the donor dye is quenched by the acceptor dye via radiation-less energy transfer. Quenching of the luminescence changes luminescence intensity and luminescence decay time. The acceptor dye reacts directly or indirectly with the analyte, thus changing its absorption values (absorption spectrum) and the rate of energy transfer. From the luminescence intensity or decay time of the donor dye inferences regarding the analyte can be made. An advantage of FRET systems lies in the fact that the expert has a choice of many known, non-luminescent indicator dyes (especially pH-sensitive absorption dyes) and that the analyte may be determined via the more sensitive luminescence measurement. Examples may be found in US 5,232,858 A (Wolfbeis et al.), in US 5,942,189 A (Wolfbeis et al.) and in Anal. Chim. Acta, 1998, 364, 143-151 (Huber et al.).

Further, the dye may comprise a DLR indicator dye system (DLR = Dual Lifetime Referencing) which comprises two luminescence dyes. In case of a CO₂ sensor according to the invention, the first dye is referred to as the pH-sensitive dye and has a short decay time. The second acts as the reference dye and has a longer decay time, e.g. in the ?s or ms range. Ideally, the two luminophores have overlapping excitation and emission spectra so that they can be excited at the same wavelength and their luminescence can be detected using the same emission window and photodetector. The phase shift of the overall luminescence obtained at a single modulation frequency of excitation light depends on the ratio of luminescence intensities of both dyes. The reference luminophore gives a constant background signal while the luminescence signal of the pH-sensitive dye depends on CO₂ partial pressure. The average phase shift directly correlates with the luminescence intensity of the pH-indicator dye and, consequently, CO2 partial pressure. DLR sensors are e.g. described in EP-B-1 000 345, DE-A-198 29 657, Liebsch et al., Anal. Chem. 73 (2001), 4354-4363); Huber et al., Anal. Chem. 73, (2001), 2097-2103, Bültzingslöwen et al., Analyst 127 (2002), 127, 1478-1483, Klimant et al., Dual Lifetime Referencing (DLR) - a New Scheme for Converting Fluorescence Intensity into a Frequency-Domain or Time-Domain Information, in New Trends in Fluorescence Spectroscopy: Application to Chemical and Life Sciences, Valeur B. & Brochon J.C. (eds.) Springer Verlag, Berlin (2001). chap. 13, 257-275, and Huber at al., Fresenius J. Anal. Chem., 368 (2000), 196-202.

Further, the invention relates to a method for determining CO₂ in a gaseous or liquid sample comprising the steps:
(a) providing a gaseous or liquid sample suspected to contain CO₂,
(b) contacting the sample with a device for determining carbon dioxide in a gaseous or liquid sample, comprising: a matrix comprising a mixture of (i) a polymer, (ii) optionally a plasticizer, (iii) an indicator comprising a pH-sensitive dye which can form an anionic dye species, (iv) a cationic species, and (v) an anionic species which is basic in relation to the protonated form of the pH-sensitive dye, wherein the cationic species is a lipophilic metal cation-ionophore complex, and
(c) determining an optical property of the pH-sensitive dye or dye system wherein the optical property (e.g., magnitude of absorbance, luminescence intensity, luminescence decay time and/or phase shift) is associated with the presence and/or the concentration or partial pressure of CO₂ in the sample.

The present invention provides a sensor device which comprises a matrix and an indicator embedded therein. The indicator may form an ion-pair comprising a metal cation-ionophore complex as a cationic species and an anion of a pH-sensitive dye. The cation and the anion are selected such that they are compatible with the matrix, i.e. the cation and the anion can form a soluble ion pair in the matrix. Preferably, the matrix comprises a homogeneous mixture of a polymer and optionally a plasticizer, an indicator comprising at least one pH-sensitive dye or a pH-sensitive dye system which can form an anionic species, and a cationic species. The polymer which is a component of the matrix is preferably a hydrophobic polymer.

CO₂ diffusing into the sensor matrix may react with water (which may be present in small amounts in the matrix) to the weak acid H₂CO₃ which protonates the anionic dye species of the pH-sensitive dye or dye system. This protonation causes a change of the optical properties (e.g. absorbance, luminescence intensity, luminescence and/or luminescence decay time) of the pH-sensitive dye or dye system. With regard to the individual reactions it is referred to the corresponding literature describing 'plastic film' CO₂ sensors as cited above. For example, an increase of the concentration or partial pressure of CO₂ in the sample may result in an increased population of protonated pH-sensitive dye molecules resulting in a corresponding change of the optical properties (e.g. decrease or increase in absorbance, luminescence intensity and/or luminescence decay time). The change in optical properties may be determined qualitatively and/or quantitatively, e.g. by visual means or by optical measurements.

The reaction of the carbon dioxide with the metal cation-ionophore complex/ dye ion-pair in the sensor matrix can be described as follows,

(1) CO₂ + H₂O + {M⁺D⁻} ↔ {M⁺HCO₃⁻} + HD

wherein {} denotes an ion-pair, M⁺ is the metal cation-ionophore complex, D-the dye anion and HD the protonated dye. The cationic metal cation-ionophore complex M⁺ acts as a counterion for the anionic dye species D⁻.

According to the invention, a second anionic species which differs from the anionic dye species is present the matrix.

The metal cation of the metal cation-ionophore complex can be added to a respective sensor formulation as hydroxide in combination with the corresponding ionophore wherein the hydroxide of the metal-cation ionophore complex {M⁺OH⁻} is generated in situ. Alternatively the metal-cation may be added as a compound selected from a basic metal salt, e.g. a bicarbonate salt (HCO₃⁻) or organic carbonate salt, preferably an alkylcarbonate salt (RCO₃⁻), a hydrogenphosphate salt (HP0₄²⁻) and a phosphate salt (PO₄³⁻) and respective organic phosphate, preferably mono- or di-alkyl phosphate salts in combination with the corresponding ionophore, wherein the respective basic compounds, e.g. {M⁺HCO₃⁻}, {M⁺RCO₃⁻}, {M⁺ PO₄³⁻} are generated in situ. The metal cation-ionophore complex with the basic anionic counterion can also be added directly to the respective sensor formulation,

The anionic species, which is the counter ion of the metal cation-ionophoer complex, is basic in relation to the protonated form of the pH-sensitive dye. Basic means in this connection that the substance present in the matrix in form of the ion pairs {M⁺OH⁻}, {M⁺HCO₃⁻} {M⁺ PO₄³⁻} etc. can remove a proton from the protonated indicator species HD, i.e., is basic over the protonated indicator species HD.

Preferred sensor formulations comprise an excess of a basic compound. The person skilled in the art will select the molar ratio of the dye molecules to metal cation-ionophore complex with the basic anionic counter anion depending on the chemical nature of the polymer, plasticizer and cationic species and the desired slopes of the sensor signal. The molar ratio of the dye molecules to metal cation-ionophore complex may range from 1:1 to 1:10000. The preferred range is 1:10 to 1:1000 and the most preferred range is 1:10 to 1:200. Equilibria involving CO₂ and excesses of basic components are summarized in Mills et al., (Anal. Chem., 64 (1992), 1583-89).

The basic character of the sensor matrix is determined by the excess amount of the metal-cation-ionophore complex with the basic counter anion added. Instead of preparation of the ion pair {M⁺D⁻} in situ in the matrix in the presence of the ionophore, it can be supplied from an ion pairing reaction of the metal cation compound with the respective dye in an appropriate solvent comprising the ionophore with which the metal cation is to be complexed at basic reaction conditions.

An important property of metal-ionophore-complexes of the present invention is that they should not interfere with the optical carbon dioxide determination. Generally speaking, the cationic species of the present sensors should not optically interfere with the determination of the optical properties of the pH-sensitive dye or pH-sensitive dye system. Preferred dyes or dye systems absorb, reflect or show luminescence in the visible light range. The preferred metal-ionophore complexes therefore should not or at least not substantially absorb, reflect or show luminescence in the visible light range (approximately between 400 nm and 800 nm). Consequently, the preferred metal-ionophore complexes of the present invention do not show luminescence or absorbance in the said range.

The cationic species according to the invention are metal cations complexed with ionophores, which are cyclic, particularly macrocyclic, or non-cyclic compounds having a molecular weight of preferably = 2000 D capable of forming ionophore-ion complexes with metal ions providing the solubility of the metal cation species in the matrix. Typically, these metal cation-ionophore complexes do not optically interfere with the indicator, the pH-sensitive dye or pH-sensitive dye system.

The ionophores may be macrocyclic compounds, i.e. compounds which have preferably at least 13 and up to 40 ring atoms. Examples of macrocyclic compounds are crownethers, which are cyclic polyethers wherein O-atoms are linked by alkylene, e.g. ethylene groups. The macrocyclic structure exhibits so-called holes in which cations can be trapped regarding to the size of the cation and the number of oxygen atoms in the macrocycle. The complex is stabilised by coordination of the cation with the free electron pairs of the oxygen atom. Further, the invention encompasses crownethers in which the O-atoms are partially or completely substituted with other heteroatoms, such as N, P or S. These compounds are designated as aza crownethers, phospha crownethers or thia crownethers. Further examples of suitable ligands are cryptands, i.e. azapolyether compounds, wherein the hole present in crown ethers is bridged, e.g. wherein two bridging nitrogen atoms are connected by one or several O-alkylene, e.g. O-ethylene containing bridges. Still, further examples are podands, which are non-cyclic analogs of crown ethers or cryptands, calixarenes, catapinates or antibiotics, e.g. peptide or macrolide antibiotics, such as valinomycin, enniatrine or nonactrine, or polyether antibiotics, such as lasalocid, monensin, nigericin or salinomycin. According to the invention, the ionophore provides lipophilic properties to the metal cation-ionophore complex.

In general, the term lipophilic refers to the ability of compounds to dissolve in fats, oils, lipids, and essentially non-polar solvents such as hexane, toluene and tetrahydrofuran. These substances are themselves lipophilic. In the present context the term "lipophilic ionophore" and "lipophilic metal cation-ionophore complex" additionally refers to the ability of these compounds to dissolve in an liquid phase consisting of an essentially non-polar polymer and optionally an hydrophobic organic plasticizer.

Specific examples of ionophores include:

| **Compound name** | **CAS-No.** | **Aldrich-Cat.No.** |
|---|---|---|
| 1-Aza-18-crown-6 | 33941-15-0 | 11382 |
| 18-crown-6 | 17455-13-9 | 274984 |
| 15-crown-5 | 33100-27-5 | 28123 |
| 4',4"(5")-Di-tert-butyldibenzo-18-crown-6 | 29471-17-8 | 34682 |
| 4',4"(5")-Di-tert-butyldicyclohexano-18-crown-6 | 28801-57-2 | 34683. |
| 4,7,13,16,21,24-Hexaoxa-1,10-diazabicyclo[8.8.8]hexacosane (Kryptofix®222) | 23978-09-8 | 52910 |
| 4,7,13,16,21-Pentaoxa-1,10-diazabicyclo[8.8.5]tricosane (Kryptofix®221) | 31364-42-8 | 76965 |
| Dibenzo-18-crown-6 | 14187-32-7 | 158399 |
| Dibenzo-15-crown-5 | 14262-60-3 | 33527 |
| Dibenzo-24-crown-8 | 14174-09-5 | 33539 |
| Benzo-18-crown-6 | 14098-24-9 | 12338 |
| Benzo-15-crown-5 | 14098-44-3 | 12335 |
| Dicyclohexano-18-crown-6 | 16069-36-6 | 158402 |
| Dicyclohexano-24-crown-8 . | 17455-23-1 | 36668 |
| Calix[4]arene | 74568-07-3 | 21262 |
| Calix[6]arene | 96107-95-8 | 21264 |
| Calix[8]arene | 82452-93-5 | 69066 |
| Calix[4]arene crown-6 | | |

An ionophore complexed metal cation may be a small-size cation, i.e. a cation having an ion radius of < 150 pm, more preferably an alkaline or an alkaline earth metal cation, particularly selected from Li, Na, K, Ca or Mg. Further, the metal cation may be a transition metal cation, which may be e.g. selected from the group consisting of Cu, Fe, Co, Ni or Ag. Furthermore, the chelated or complexed metal cation may be a big-size metal cation (= 150pm), e.g. Rb⁺or Cs⁺.

A particular advantage of the preferred cationic species of the present invention, namely metal cation-ionophore complexes of alkaline or alkaline earth metal cations, is their higher stability against redox active compounds, e. g. oxidants, compared to transition metal salt complexes (as e. g. proposed in EP 0 837 327) or against degradation compared to the quaternary ammonium ions proposed by Mills et al. (cf. e. g. Anal. Chem. 64 (1992) 1388 and other references cited *supra).*

Ion pairs of metal cation-ionophore complexes and dye anions show also an improved solubility.

The pH-sensitive dye or dye system comprises a compound which can be protonated by reaction with an acidic reacting component, e.g. carbon dioxide, in the presence of H₂O.

Examples of suitable pH-sensitive dyes are dyes with acidic reacting groups such as phenolic groups, and/or carboxylic acid groups with pK values in watery environments in the range of 5 to 10, preferably of 7 to 9. The pK value is the negative log of the thermodynamic equilibrium constant of the deprotonation reaction.

pH-sensitive dyes exist in at least two forms, at least one protonated species A and at least one deprotonated species B, the two species and the proton (i.e., - H⁺ or H₃O⁺ respectively) being in thermodynamic equilibrium (HD ↔ D⁻+ H⁺).

In case of the CO₂ sensor of the present invention, the pH-sensitive dye is reversibly protonated through direct or indirect interaction with CO₂, i.e., according to the equilibrium reaction shown in equation (1). Deprotonation decreases the positive electric charge (e.g., from (0 to -1, -1 to -2, etc) of the dye species, thereby generating an anionic species or increasing the charge of an already anionic species. The generated anionic charge has to be compensated by the positive charge of a cationic species. In case of the present sensor, it is required that both, the cationic species and the ion-pair (consisting of the anionic dye species and the cationic species) are soluble in the matrix.

Luminescent pH-sensitive dyes can be selected from pH-sensitive hydroxypyrenes, fluoresceines, rhodamines, umbelliferones, coumarins, and derivatives like alkyl esters or amides of these. Particularly preferred luminescent dyes are 7-hydroxycoumarin-3-carboxylic acid 8-hydroxypyrene- 1,3,6-trisulphonate, 5(6)-carboxyfluorescein, 5(6)-carboxy naphthofluorescein (C652, Invitrogen), carboxy SNARF-1 (C1270, Invitrogen), carboxy SNARF-4F (S23920, Invitrogen), carboxy SNARF-5F (S23922, Invitrogen), carboxy SNAFL-1 (C1255, Invitrogen), 2'-chlorofluorescein, 2'-chloro-7'-hexylfluorescein, 2',7'-dichlorofluorescein, 2'-chlorofluorescein hexylester, 2'-chlorofluorescein octadecylester and 2',7'-dichlorofluorescein octadecylester in protonated and/or deprotonated forms.

Absorbance-based pH-sensitive dyes, can be selected from pH-sensitive, triphenylmethane dyes, azo dyes, etc.

Preferred absorbance-based pH-sensitive dyes include bromothymol blue, thymol blue, m-cresol purple, cresol red, phenol red, xylenol blue and brilliant yellow.

Additional pH-sensitive dyes useful in the present invention may be found in A. Mills and S. Hodgen, ,Fluorescent Carbon Dioxide Sensors' in ,Topics in Fluorescence Spectroscopy', Volume 9, Advanced Concepts in Fluorescence Sensing, Part A: Small Molecule Sensing, C. D. Geddes and J. R. Lakowicz (eds.), Springer, 2005, p. 119-161.

The anionic form of the indicator dyes may be lipophilized in order to increase solubility in the matrix via ion pair formation. Water soluble sodium salts of the dyes may be converted to the desired metal cation ion pair through extraction into organic solvents with the appropriate metal cation crown ether complex from aqueous solution.

The sensor according to the invention may be in any suitable form, e.g. the sensor matrix comprising the indicator may be present as particle, as a substrate-based coating or a compact substrate. Preferably, the sensor matrix is provided as a film on a substrate, e.g. an opaque or transparent inert substrate, preferably a plastic substrate. The film preferably has a thickness of 1-200 ?m, preferably of 10-100 ?m. The polymer matrix is preferably transparent. Further, the sensor matrix should be hydrolytically stable and be permeable to carbon dioxide.

The sensor of the present invention is preferably substantially dry, i.e. the sensor matrix has a low water content, e.g. a water content of about 10% (w/ w) or less, preferably of about 5% (w/w) or less. Preferred is water content of about 0.01 % (w/w) to about 1 % (w/w).

The sensor matrix preferably comprises a polymer which is selected from the group of polyvinyl-based polymers, acryl-based polymers, styrene-based polymers, cellulose-based polymers, polyurethane-based polymers polyesters-based polymers, or polysiloxane-based polymers or combinations thereof. Preferably, the polymer is an essentially hydrophobic polymer.

Additionally the polymer can be based on polyesters or polysiloxanes, e.g. sol-gels derived from tetramethoxysilane or tetraethoxysilane precursors, polydimethylsiloxane and other organically modified siloxanes.

Especially preferred polymers are cellulose-based polymers, such as ethyl cellulose, methyl cellulose, amino cellulose etc., polyurethanes, polysiloxanes, e.g. sol-gels derived from tetramethoxysilane or tetraethoxysilane precursors, polydimethylsiloxane and other organically modified siloxanes, silicon rubber etc.

Further, the sensor matrix may additionally comprise a plasticizer. Suitable plasticizers are e.g. hydrophobic plasticizers including alkyltriesters of phosphoric acid, esters of carboxylic acids, e.g. those with secondary or tertiary alcohols, sulphamides etc. Specific examples of plasticizers are dioctylsebacate (DOS), trioctylphosphate (TOP), cyanophenyloctylester (CPOE), nitrophenyloctylester (NPOE), 2-(octyloxy)benzonitrile (OBN), tributylphosphate (TBP), etc.

The sensor and the method of the invention are suitable for determining the concentration or partial pressure of CO₂ in gaseous or liquid samples, e.g. in industrial or medical applications. For example, the present invention is suitable for determining the carbon dioxide content in blood or respiratory gases.

The method of the present invention is based on the measurement of the optical properties of an indicator present in the sensor matrix of an optical sensor.

A preferred application is the determination of CO₂ in medical diagnostic methods, e.g. the determination of CO₂ in body fluids, particularly for determining the partial pressure of CO₂ (pCO₂) either alone or in combination with other parameters such as partial pressure of oxygen (pO₂), pH and electrolytes such as K⁺, Na⁺, Ca²⁺ and/or Cl⁻. Further preferred applications are medical or non-medical biotechnology, particularly analytics, monitoring, quality control, regulation and/or optimization of biotechnological processes. Particularly preferred applications are measurements of CO₂ in the central nervous system, e.g. in the brain, for blood or respiratory gas analysis, transcutaneous analysis diagnosis of gases and liquids in human and veterinary medicine, measurement of CO₂ in cell culture growth, in fermentation or in monitoring metabolic activity, measurement of CO₂ in drug screening, in food or beverage packing, or measurement of CO₂ in environmental samples, e.g. fresh water, sea water or waste water samples.

Consequently, a further aspect of the present invention is the use of the sensor for determining carbon dioxide. In particular, the use of the sensor for determining carbon dioxide encompasses the determination of CO₂ in blood or respiratory gases, in biotechnological processes, in cell culture growth, in fermentation or in monitoring metabolic activity, especially for controlling and/or optimizing processes in biotechnological applications, in drug screening, in food or beverage packaging, for controlling process quality or for optimisation of processes, in environmental samples, in fresh water, sea water or waste water.

The sensor may be a luminescent optical sensor, e.g. an optode as described in Ann. Biol. Clin. 2003, 61: 183-191 which is herein incorporated by reference. The sensor can also be an absorbance based optical sensor, a FRET-based optical-sensor or a DLR-based optical sensor. Absorbance based sensors are e.g. described by Mills A. and Chang Q. (Sensors and Actuators B21 (1994), 83-89) and Mills et al. (Sensors and Actuators B38-39 , (1997), 419-425). FRET-based sensors are e.g. described by Liebsch et al. (Applied Spectroscopy 54/4 (2000), 548-559) and Neurauter et al. (Analytica Chimica Acta 382 (1999), 67-75). DLR-based sensors are e.g. described by Bültzingslöwen C. von (Analyst 127 (2002), 1478-1483) and Borisov et al. (Applied Spectroscopy 60/10 (2006), 1167-1173).

A preferred sensor comprises a sensor matrix comprising the indicator as described above, e.g. a sensing layer on an optical transparent substrate, e.g. a polyester foil.

The sensing layer is preferably covered with an opaque, e.g. black or white overcoat-layer which is permeable to the acidic compound to be determined. The overcoat layer acts as a precaution to protect the sensing layer from optical and/or chemical interferences with components of the sample. The bottom part of the substrate may be covered with an optical transmissive adhesive layer for attachment in a cartridge.

Further, the device preferably comprises the sensor as describes above, a light source, e.g. an LED, for irradiating the sensing layer with light capable of exciting the pH-sensitive dye. Furthermore, the device preferably comprises means for detecting emission light from the indicator dye, e.g. a photodiode. The emission light has preferably a higher wavelength than the excitation light.

The sensor matrix of the present invention may be fabricated by conventional techniques, e.g. casting techniques, wherein the components of the matrix, i.e. polymer, cationic species, i.e. metal cation-ionophore complex, pH-sensitive dye and optionally plasticizer, are dissolved in an organic or organic-aqueous solvent and cast onto a suitable substrate.

Cations such as Li⁺, Na⁺, K⁺ are preferably added as complexes with ionophores. In these cases, the counterions are preferably independently selected from OH-, an anion of pH-sensitive dye, and/or an organic anion, particularly of an organic phosphate or carbonate (e.g., alkylphosphate and/or alkylcarbonate).

Furthermore, the present invention is to be explained in greater detail be the Figures and Examples hereinbelow.

### Figure Legends

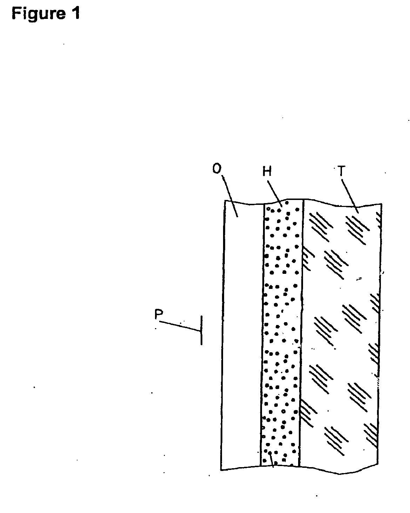

**Figure 1****:** A schematic description of CO₂ sensor according to the invention. The sensor comprises a support (T), an indicator layer (H) and a light-blocking layer (O). On the support (T), the indicator layer (H) is provided directly or through an adhesive layer. The light-blocking layer (O) can optionally be provided on top of the indicator layer (H). The light-blocking layer (O) is a preferably gas-permeable and ion-impermeable layer wherein light-absorptive or light-reflecting (called "light-blocking" collectively) particles are dispersed. The light-blocking layer (O) protects from any optical interferences with the sample (P).
**Figure 2****:** Time-trace of the dry CO₂ sensor involving the use of a solubilized K⁺ as metal cation. Fluorescence intensity was measured in humidified gas with carbon dioxide content of 0, 1, 3, 5, 10, 20, 30, 50 and 100%, respectively.
**Figure 3****:** Calibration plot of the sensor shown in Fig. 2 involving the use of (K⁺-crown 6) as solubilized metal cation. The light line represents a Boltzman-Fit for a single measurement with a correlation coefficient of R² = 0.9997.

### Example

### Example 1: Sensor involving the use of a solubilized metal cation

General formulation: Ethylcellulose/HPTS Tri (K-crown 6) salt/ (Z-crown 6) hydroxide/white Teflon overcoat (TiO₂ in Teflon AF)
Solution I: 10 mg HPTS
   34.7 mg 18-crown-6
   955.3 mg methanol
Solution II: 15.0 mg KOH
   157.9 mg 18-crown-6 (Aldrich 34682)
   1326.7 mg methanol

### Fabrication of optical sensors

To 1 g of a 10% w/w solution of ethylcellulose in toluene/ethanol (4/1 v/v) 26 mg of solution I were added. 56.2 mg of solution II were added to this mixture and the casting solution was mixed thoroughly. The respective homogeneous coating solution was coated on a 125 µm polyester foil (Mylar foil, Goodfellow ES301425) with a wet film thickness of 150 µm using a knife coating device (Zehntner ZAA2300). The dried sensor film was translucent. Additionally, the sensor film was coated with a white Teflon layer of 150 µm wet film thickness for signal enhancement.

Disks of 5 mm diameter were punched out and fixed at the distal end of a bifurcated Y-type optical light guide. The other connectors of the light guide were connected to a blue LED (Yₘₐₓ = 470 nm) and to a PMT, respectively, both controlled with a dual lock-in amplifier (Stanford Research SR830). The optical setup included a blue excitation filter (BG12, Schott AG) and an orange emission filter (OG530, Schott AG). The sensor containing end of the light guide was placed in a self-made measuring chamber located in a water bath of 20±1?C. The inlet opening of the chamber was connected to a specially designed gas mixing device allowing gas flow of humidified gases of different carbon dioxide content at a flow rate of 1 L/min through the chamber.

### Results:

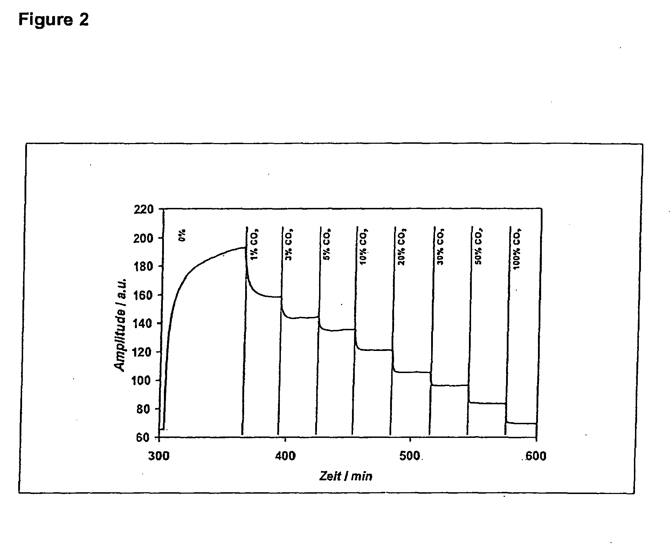

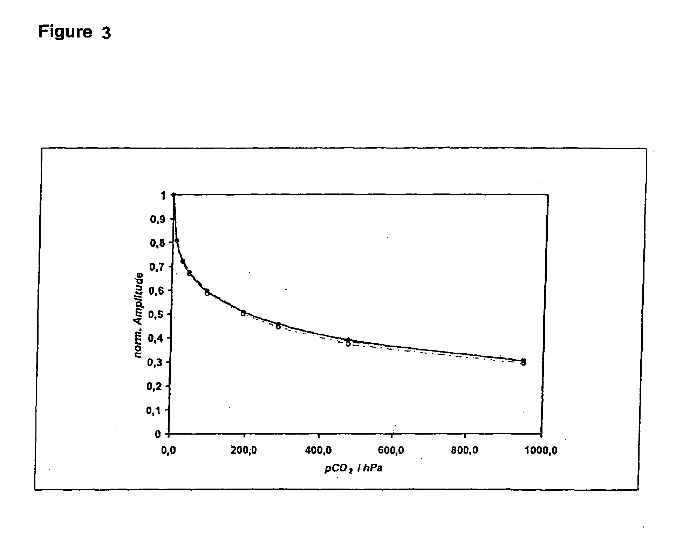

In Figure 2 the response of the sensor using a solubilized metal cation is displayed upon exposure to gases of different carbon dioxide content of 0, 1, 3, 5, 10, 20, 30, 50 and 100%, respectively. The calibration plot obtained is shown in Figure 3. It is obvious that the solubilization of the metal cation is feasible for the production of carbon dioxide sensitive materials. The homogeneity and transparency of the coated films shows good solubility of the ion-paired dye and complexed metal cation, and thus good compatibility of the components.

## Claims

1. An optical sensor for determining carbon dioxide in a gaseous or liquid sample, comprising:
a matrix comprising a mixture of
(i) a polymer,
(ii) optionally a plasticizer,
(iii) an indicator comprising a pH-sensitive dye which can form an anionic dye species,
(iv) a cationic species, and
(v) an anionic species which is basic in relation to the protonated form of the pH-sensitive dye,
wherein the cationic species is a lipophilic metal cation-ionophore complex.

2. An optical sensor for determining carbon dioxide in a gaseous or liquid sample, comprising:
a matrix comprising a mixture of
(i) a polymer,
(ii) optionally a plasticizer,
(iii) an indicator comprising a pH-sensitive dye which can form an anionic dye species,
(iv) a cationic species, and
(v) an anionic species which is basic in relation to the protonated form of the pH-sensitive dye,
wherein the cationic species is a lipophilic, metal cation-ionophore complex and wherein the cationic species acts as a counterion for the anionic dye species.

3. The sensor of any one of claim 1 or 2,
wherein the cationic species is a metal cation-ionophore complex of an alkaline metal cation, particularly selected from Li⁺, Na⁺, K⁺, Rb⁺ or Cs⁺.

4. The sensor of any one of claims 1-3,
wherein the ionophore is selected from the group consisting of podands, crownethers, cryptands, calixarenes and valinomycins.

5. The sensor according to any one of the claims 1-4,
wherein the pH active part of the dye or dye system is present in both protonated and deprotonated forms, wherein the ratio of protonated and deprotonated forms depends on pCO₂ of the gaseous or liquid sample, and wherein the deprotonated form and the cationic species form an ion pair.

6. The sensor of any one of claims 1-5,
wherein the pH-sensitive dye or dye system is dissolved in the matrix.

7. The sensor of any one of claims 1-6,
wherein the pH-sensitive dye or dye system is bound to the polymer.

8. The sensor of any one of claims 1-6,
wherein the pH-sensitive dye or dye system is bound to the plasticizer.

9. The sensor of any one of claims 1-6,
wherein the pH-sensitive dye or dye system is bound to the surface of particles dispersed in the polymer matrix.

10. The sensor of any one of claims 1-9,
wherein the pH-sensitive dye is selected from the group consisting of fluorescein, umbelliferone, 7-hydroxycoumarin-3-carboxylic acid, bromothymol blue and 8-hydroxypyrene-1,3,6-trisulphonate, 5(6)-carboxyfluorescein, 5(6)-carboxy naphthopfluorescein, carboxy SNARF-1, carboxy SNARF-4F, carboxy SNARF-5F, carboxy SNAFL-1 and combinations thereof.

11. The sensor of any one of claims 1-10,
wherein the matrix comprises counteranions of the cationic species selected from the group of hydroxide, bicarbonate, organic carbonate and organic phosphate.

12. The sensor of any one of claims 1-11,
wherein the matrix is in the form of a film on a substrate.

13. The sensor of any one of claims 1-12, wherein the matrix is in form of particles dispersed within an inert film on a substrate.

14. The sensor of any one of claims 1-13,
wherein the matrix comprises a vinyl-based polymer, an acrylic-based polymer, a styrene-based polymer, a cellulose-based polymer, a polyurethane-based polymer or a combination thereof.

15. The sensor of any one of claims 1-13,
wherein the matrix comprises a polymer based on polysiloxanes, e.g. sol-gels derived from tetramethoxysilane or tetraethoxysilane precursors, polydimethylsiloxane and other organically modified siloxanes (ormosils), a polymers including a fluoropolymer, e.g., polytetrafluoroethylene, a polydiene, a polyester or a combination thereof.

16. The sensor of claim 14,
wherein the polymer matrix comprises ethyl cellulose.

17. The sensor of any one of claims 1-16,
wherein the matrix has a water content of 10% (w/w) or less, preferably of 5% (w/w) or less.

18. The sensor of any one of claims 1-17,
wherein the molar ratio of the dye molecules to metal cation-ionophore complex is in the range of 1:1 to 1:10000, preferably in the range of 1:10 to 1:1000, most preferably in the range of 1:10 to 1:200.

19. Use of a sensor of any one of claims 1-18 for determining carbon dioxide.

20. A method for determining carbon dioxide in a gaseous or liquid sample comprising the steps:
(a) providing a gaseous or liquid sample suspected to contain carbon dioxide,
(b) contacting the sample with the sensor of any one of claims 1-18, and
(c) determining an optical property of the sensor, wherein the optical property is associated with the presence and/or amount of carbon dioxide in the sample.
